# EUROPÄISCHE PATENTSCHRIFT

(11) **EP 1 575 427 B1**
(45) Veröffentlichungstag und Bekanntmachung des Hinweises auf die Patenterteilung: **26.09.2007**
(21) Anmeldenummer: 04790640.9
(22) Anmeldetag: 19.10.2004
(51) Int. Cl.: A61B 5/16

(54) **VORRICHTUNG ZUR VITALITÄTSDIAGNOSTIK**
DEVICE FOR VITALITY DIAGNOSIS
DISPOSITIF DE DIAGNOSTIC DE VITALITE

(30) Priorität: 21.10.2003 DE 20316285 U
(43) Veröffentlichungstag der Anmeldung: 21.09.2005
(73) Patentinhaber: Gruber, Richard, Dr. med., 86825 Bad Wörishofen (DE)
(72) Erfinder: Gruber, Richard, Dr. med., 86825 Bad Wörishofen (DE)
(74) Vertreter: Behrmann, Niels
(86) Internationale Anmeldenummer: PCT/EP2004/011820
(87) Internationale Veröffentlichungsnummer: WO 2005/039414

(56) Entgegenhaltungen:
- WO-A-02/41108
- DE-A1- 19 544 136
- DE-U1- 20 015 449
- FR-A- 2 617 730
- US-A- 5 904 639
- US-A1- 2003 055 322
- US-A1- 2003 078 501

## Beschreibung

Die vorliegende Erfindung betrifft eine Vorrichtung zur Vitalitätsdiagnostik, wie sie aus dem deutschen Gebrauchsmuster 200 15 449 bekannt ist.

Eine derartige Vorrichtung ermöglicht es, durch das Durchführen einer Mehrzahl von Einzeltests das sogenannte biologische oder funktionale Alter einer Testperson zu erfassen, indem altersabhängige Organfunktionen sowie andere alterungsrelevante bzw. physische Parameter erfasst, nach Alter, Geschlecht usw. klassifiziert und durch Zuordnung zu Bezugsgruppen im Hinblick auf eine relative Leistungsfähigkeit bezogen auf die Bezugsgruppen aufbereitet werden.

Es hat sich dabei herausgestellt, dass gerade die durch die Vorrichtung nach dem Stand der Technik ermöglichte Vielzahl von Tests eine holistische Betrachtung der physischen Leistungsfähigkeit der Testperson ermöglichen, so dass insbesondere auch seriösen wissenschaftlichen Anforderungen bei der Messung und Auswertung Rechnung getragen werden kann und so ein Werkzeug entsteht, welches hinsichtlich der demographischen Entwicklungen wertvolle Dienste zur Förderung des Gesundheitsbewusstseins sowie zur Prävention altersbedingter Probleme leisten kann.

Die aus dem Stand der Technik gemäß DE 200 15 449 bekannte Vorrichtung macht sich dabei zur Anzeige sowie zur Erfassung von Eingabereaktionen der Testperson einen berührungsempfindlichen Bildschirm zunutze, mit welchem Einfachheit in Bedienung und Darstellung mit hoher Zuverlässigkeit und Betriebssicherheit sowie einfacher Fertigbarkeit kombinierbar sind; zusätzlich werden weitere, speziellere Diagnostikfunktionen mittels individuell ausgebildeter, bevorzugt drahtlos angebundener Sensoreinheiten (welche dann in der notwendigen Weise durch die Testperson manuell gehandhabt werden) realisiert.

Allerdings ist eine derartige, aus dem Stand der Technik bekannte Vorrichtung insbesondere im Hinblick auf Einfachheit beim Transport und Aufbau (gerade im Hinblick auf mobile Verwendungen bzw. Installation und Inbetriebnahme durch technische Laien) nicht unproblematisch; zum einen verlangt das voluminöse, durch den berührungsempfindlichen Bildschirm erforderliche Pultgehäuse nicht unbeträchtlichen Platz- und damit Transportaufwand, auch sorgt die Mehrzahl der zusätzlich vorgesehenen, manuell betätigbaren Sensoreinheiten für potentielle Verwirrung bei ungeschulten Bedienern sowie verursacht zusätzlichen Platzaufwand für Transport und im Gebrauch (bei der aus dem Stand der Technik bekannten Vorrichtungen sind insgesamt vier derartige manuell betätigbare Sensoreinheiten senkrecht auf dem Pultgehäuse gelagert).

Aufgabe der vorliegenden Erfindung ist es daher, eine derartige, aus dem Stand der Technik bekannte und als gattungsbildend vorausgesetzte Vorrichtung im Hinblick auf ihre Mobilität und Portabilität zu vereinfachen, insbesondere eine Vorrichtung zu schaffen, welche zumindest in einem Transportzustand eindeutig geringeres Transportvolumen erfordert, und welche zudem im Hinblick auf durch die manuell handhabbaren Sensoreinheiten geringeren Handhabungs-, Transport- und Lagerungsaufwand verursacht.

Die Aufgabe wird durch die Vorrichtung nach dem Hauptanspruch gelöst.

Genauer gesagt ist vorteilhaft vorgesehen, den berührungs- und/oder druckempfindlichen Bildschirm so klappbar auszugestalten, dass dieser in einer Betriebsposition bevorzugt winkelverstellbar in einem für die Betrachtung bzw. Betätigung geeigneten Winkel auf der Gehäuseeinheit der Datenverarbeitungsvorrichtung aufsitzt und weiter bevorzugt in dieser Stellung (bzw. mehreren Stellungen) rastend befestigbar ist. Durch Umklappen, insbesondere mittels eines weiterbildungsgemäß vorgesehenen Bügels, lässt sich das Bildschirmgehäuse dann auf die Gehäuseeinheit falten, so dass eine flache, für einen Transport günstig geeignete und ein geringes Transportvolumen benötigende Einheit entsteht, wobei in dieser Konfiguration zusätzlich ein wirksamer Schutz der Bildschirmoberfläche durch Aufliegen auf einer Flachseite der Gehäuseeinheit vorliegt.

Im Rahmen der vorliegenden Beschreibung die mindestens eine Betätigungs- und Sensoreinrichtung versenkbar in der Gehäuseeinheit vorzusehen (alternativ ist dies auch eine Weiterbildung der unabhängigen Lösung). Vorteilhaft wird auf diese Weise nicht nur eine Transportsicherung für die empfindliche Betätigungs- und Sensoreinrichtung erhalten, auch ermöglicht es diese Realisierungsform der Erfindung, durch entsprechendes Ausklappen eine Betriebs- bzw. Zugriffsposition für die Testperson anzubieten. Im eingeklappten Zustand ist zudem der Platzbedarf der Gesamtanordnung minimiert.

Gemäß der Erfindung ist vorgesehen, ein bevorzugt zylindrisch ausgebildetes Gehäuse für mindestens eine Betätigungs- und Sensoreinrichtung mit einer Mehrzahl von diagnostischen Funktionalitäten auszubilden. So lässt sich nicht nur effektiv die Zahl der benötigten Betätigungs- und Sensoreinrichtungen reduzieren, auch wird der Bedienungs- (und damit der Schulungs- und Erklärungs-)aufwand für die Gesamtanordnung reduziert, so dass im Ergebnis die Bedienungsfreundlichkeit der Vorrichtung gesteigert werden kann. In der konkreten Realisierung dieser Lösung hat es sich dabei als bevorzugt herausgestellt, die auf das visuelle Darstellen eines durch die Testperson zu betrachtenden optischen Musters gerichtete Funktionalität mit der Funktionalität zur Erfassung der Handkraft zu kombinieren. Nicht nur lässt sich die entsprechende mechanische Ausrüstung der so entstehenden bifunktionalen Sensoreinheit günstig bewerkstelligen, auch entsteht vorteilhaft die Möglichkeit zur mehrfachen Nutzung von am Gehäuse für die Betätigungs- und Sensoreinrichtung vorgesehenen Bedienungselementen (etwa der weiterbildungsgemäß vorgesehenen Bügeleinheit, die einerseits zur Kraftaufnahme und - einleitung in entsprechend zugeordnete Drucksensoren vorgesehen ist, andererseits jedoch auch, im Rahmen der weiteren visuellen Testfunktionalität, von der Bedienperson zur vornahme von Einstellungen oder zur Abgabe von Bestätigungssignalen betätigt werden kann).

Es hat sich zudem in der praktischen Realisierung der Erfindung als bevorzugt herausgestellt, die drahtlose Anbindung der Betätigungs- und Sensoreinrichtungen mittels Funksignalen durchzuführen. Nicht nur wird hierdurch, etwa gegenüber einer Infrarot-Datenverbindung, eine größere räumliche Flexibilität erreicht, auch lässt sich mit Hilfe günstig beschaffbarer, die jeweils benötigten Funkprotokolle realisierender Funkmodule die drahtlose Anbindung mit geringem Aufwand und kostengünstig realisieren.

Im Ergebnis entsteht durch die im Rahmen der vorliegenden Erfindung getroffenen Maßnahmen eine signifikante Verbesserung der als gattungsbildend vorausgesetzten Technologie, welche sich insbesondere im Hinblick auf den Testbetrieb, aber auch für häufig notwendigen Transport bei mobiler Nutzung, als nützlich erweisen. Nicht zuletzt steht zu erwarten, dass durch die vorliegenden Lösungsmaßnahmen noch weiteren Zielgruppen die Vorteile der mit der erfindungsgemäßen Vorrichtung ermöglichten diagnostischen Verfahren erschlossen werden können.

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in
- Fig. 1: eine schräge Perspektivansicht der erfindungsgemäßen Vorrichtung zur Vitalitätsdiagnostik gemäß einer ersten, bevorzugten Ausführungsform im Betriebszustand mit in vorbestimmtem Winkel gehaltenem Bildschirmgehäuse und zur Aufnahme durch die Bedienperson aufrechtstehendem Paar von Betätigungs- und Sensoreinrichtungen;
- Fig. 2: eine Seitenansicht gemäß Fig. 1 mit schematisch verdeutlichter Klappmöglichkeit des Bildschirmgehäuses und einer in der Gehäuseeinheit der Datenverarbeitungsvorrichtung eingeklappt teilweise verborgenen Betätigungs- und Sensoreinrichtung;
- Fig. 3: eine Ansicht der Vorrichtung gemäß Fig. 1, Fig. 2 in zusammengeklapptem Lagerungs- und Transportzustand mit dem Bildschirmgehäuse bildschirmseitig auf einer oberen Flachseite der Gehäuseeinheit aufliegendem Bildschirm im Faltzustand und beidseitig eingeklapptem Paar der Betätigungs- und Sensoreinrichtungen und
- Fig. 4: einer seitlichen Detailansicht einer bifunktionalen Betätigungs- und Sensoreinrichtung gemäß der Ausführungsform nach Fig. 1 bis Fig. 3.

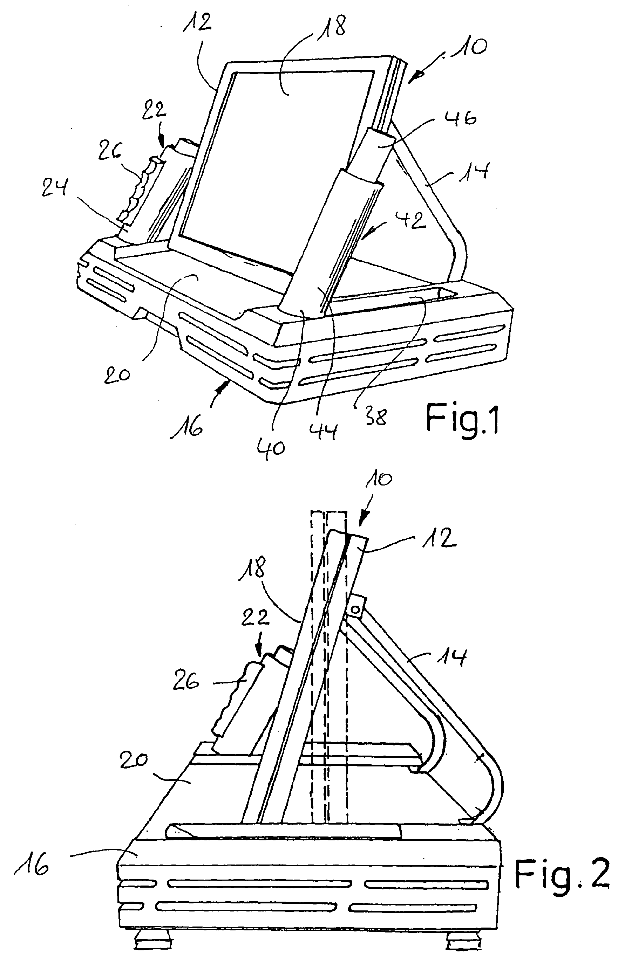

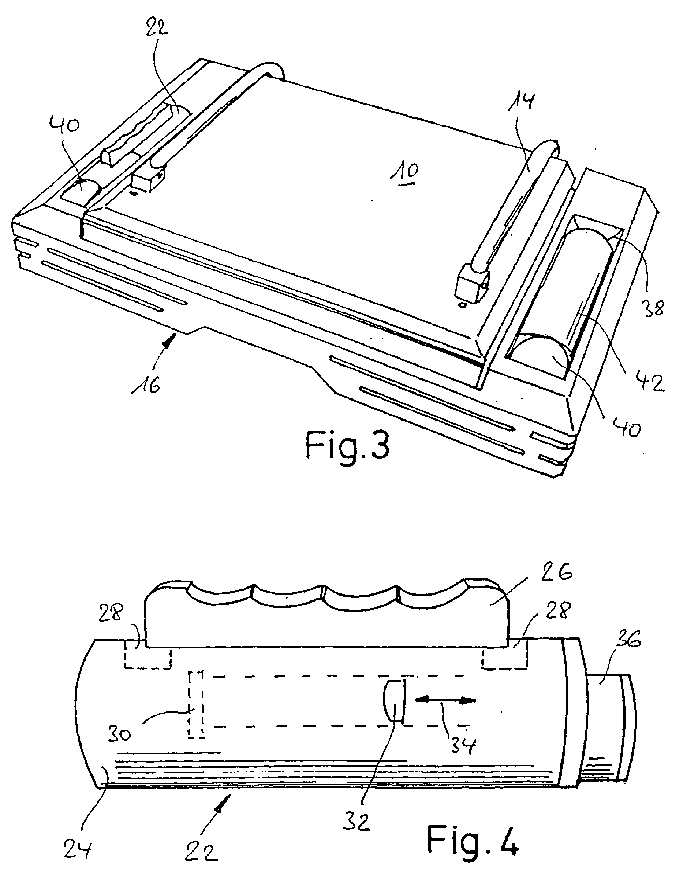

Die Fig. 1 verdeutlicht den Betriebszustand der erfindungsgemäßen Vorrichtung zur Vitalitätsdiagnostik gemäß einer bevorzugten Ausführungsform. Eine ansonsten bekannte, berührungs- und druckempfindliche Bildschirmeinheit 10 ist in einem zugehörigen Bildschirmgehäuse 12 schwenkbar an einem bezogen auf die Breite beidseitig angreifenden Metallbügel 14 so an einem flachen, die Datenverarbeitungsvorrichtung aufnehmenden Gehäuse (Gehäuseeinheit) 16 angelenkt, dass die Bildschirmeinheit 10 von dem in Fig. 1 gezeigten Betriebszustand in einen Lager- und Ruhezustand, wie in Fig. 3 gezeigt, gefaltet werden kann, wobei, vergleiche die Fig. 2, zu diesem Zweck die Bildschirmeinheit 10 in Richtung auf das hintere Ende des Gehäuses 10 verschwenkt wird und, vergleiche die Draufsicht der Fig. 3, dann so vom abgewinkelten Bügel 14 umgeben ist, dass der Platzbedarf minimiert ist und der Bügel 14, über seine Dicke hinaus, nicht nach oben vorsteht.

Wie in der Fig. 3 gut erkennbar ist, ruht in dieser Transportposition die Bildschirm- (Darstellungs-)seite 18 der Bildschirmeinheit, auf welcher dann geeignete Bedienknöpfe, Erläuterungstexte usw. gezeigt und zur Betätigung angeboten werden, auf einer Gehäuseflachseite 20 der Gehäuseeinheit 16, so dass insoweit ein Schutz vor schädlichen Beeinträchtigungen, Verschmutzungen usw. gegeben ist.

Ferner verdeutlichen die Fig. 1 bis 3 die Lagerung (Aufnahme) von im Rahmen der Erfindung bifunktionalen Betätigungs- und Sensoreinrichtungen am bzw. im Gehäuse 16. Genauer gesagt ist zunächst im dargestellten Ausführungsbeispiel einends seitlich der Bildschirmeinheit 16 eine erste Betätigungs- und Sensoreinrichtung 22 teilweise versenkt und klappbar gelagert, welche für den bestimmungsgemäßen Zweck der Vitalitätsdiagnose zum einen eine Messung der Augenakkommodation der Testpersonen ermöglicht, zum anderen zum Messen der Handkraft der Testpersonen ausgebildet ist. Zu diesem Zweck, vergleiche Fig. 4 sowie die darin mit gestrichelten Linien angedeuteten internen Funktionskomponenten der ersten Betätigungs- und Sensoreinrichtung, ist außerhalb eines zylindrischen Gehäuses 24 ein bügelartiger Griff 26 so befestigt, dass durch manuelles Greifen des Gehäuses und Umgreifen des geeignete Fingermulden aufweisenden Griffes 26 eine Fingerkraft auf den Griff aufbringbar ist und, durch geeignete Lagerung des Griffes am Gehäuse 24, eine Kraft auf ein Paar von im Gehäuseinneren unterhalb der Griffenden angeordneten Drucksensoren 28 ausübt wird. Deren Sensorsignal wird in ansonsten bekannter, nicht gezeigter Weise mittels einer im Inneren des zylindrischen Gehäuses 24 vorgesehenen Auswerte- und Funkelektronik ausgewertet und dann drahtlos zur Aufbereitung und Weiterverarbeitung an die im Gehäuse 16 vorgesehene Datenverarbeitungsvorrichtung gesendet.

Zusätzlich enthält die erste Betätigungs- und Sensoreinrichtung zur Realisierung einer Augenakkommodationsmessung eine lediglich schematisch und gestrichelt dargestellte, mittels in Kreisform angeordneter Leuchtsegmente (nicht dargestellt) realisierte Leuchteinheit 30, welche, ebenso über die gehäuseintern vorgesehene Signalelektronik angesteuert, vorbestimmte Leuchtmuster durch selektive Aktivierung der Leuchtsegmente erzeugt. Der Leuchteinheit 30 zugeordnet ist eine mittels eines axial im Gehäuse 24 geführten Schlittens bewegbare Linse 32, welche durch die Bedienperson zum Zweck der Scharfstellung in Pfeilrichtung 34 verschoben werden kann, wenn die Bedienperson durch ein einends am zylindrischen Gehäuse 24 vorgesehenes Okular 36 blickt. Genauer gesagt kann er entweder durch Betätigung des Griffes 26 (und Aufbringen einer Druckkraft auf einen der beiden Drucksensoren 28), oder aber durch Betätigen entsprechend vorgesehener Betätigungsfelder auf der Bildschirmeinheit 10, eine seiner subjektiven visuellen Situation entsprechende Scharfstellung vornehmen, so dass in der ansonsten bekannten Weise durch Steuerung der Datenverarbeitungsvorrichtung der Augenakkommodationstest durchgeführt werden kann. Im Gegensatz zu bekannten Ziffern- bzw. Zahlendarstellungen wird jedoch die erstmals hier mittels der kreisförmig angeordneten Leuchtsegmente ausgebildete Leuchteinheit (bei welcher dann im Testbetrieb selektiv einzelne oder mehrere Segmente ausgeblendet werden) in überaus wirksamer, diagnostisch valider Weise die gewünschte Augenfunktion gemessen.

Wie durch Vergleich der Fig. 1 bzw. 2 (hier ist jeweils die erste Betätigungs- bzw. Sensoreinheit in einem aufgerichteten, zur Abnahme und Bedienung durch die Testperson vorgesehenen Zustand gezeigt) mit der Fig. 3 erkennbar ist, weist das Gehäuse 16 beidseits der Bildschirmeinheit 10 eine muldenförmige Einformung 38 auf, in welche, durch ein geeignetes Aufsatz- und Klappstück 40, welches einends in der Mulde gelagert ist, die Betätigungs- und Sensoreinheit von einem versenkten Lagerungszustand (genauer gesagt handelt es sich hier lediglich um einen teilweise versenkten Zustand) in den Bereitschaftszustand gemäß Fig. 1, Fig. 2 verschwenkt werden kann.

Dieses Lagerungs- und Bedienprinzip gilt analog für die zweite, aus der Betrachtungssicht auf die Bildschirmeinheit 10 rechts von dieser angeordnete Betätigungs- und Sensoreinheit 42. Auch diese ist im beschriebenen Ausführungsbeispiel als bifunktionale Vorrichtung zur Realisierung von zwei Vitalitätsdiagnostik-Funktionen ausgebildet, und zwar zum einen für einen Lungenfunktionstest, zum anderen für einen Vibrationstest, um die Vibrationsempfindlichkeit der das zylindrische Gehäuse 44 der zweiten Betätigungs- und Sensoreinheit 42 umgreifenden Hand der Testperson zu messen. Genauer gesagt ist im Gehäuse 44 in der Art eines ansonsten bekannten Staurohrs eine Drucksensoreinheit (nicht gezeigt) vorgesehen, welche sowohl vor als auch hinter einer den Standeffekt bewirkenden Verengung einen durch Einblasen in ein Mundstück 46 des Gehäuses 44 erzeugten Luftstrom (durch Differenzdruck vor und hinter der Verengung im Gehäuse) misst. Aus diesem Differenzdruck kann dann auf Lungenvolumen und andere vitalitätsrelevante Parameter der die zweite Betätigungs- und Sensoreinheit 42 handhabenden Testperson durch geeignete Auswertung geschlossen werden.

Als zweite Funktionalität im Gehäuse 44 ist der Gehäuseinnenwand unmittelbar benachbart ein elektromechanischer Vibrationsgeber vorgesehen (typischerweise als Magnetspule mit Kern realisiert), welcher, geeignet durch die Datenverarbeitungsvorrichtung über eine nicht gezeigte Funkstrecke und Treiberelektronik in der zweiten Betätigungs- und Sensoreinheit ansteuerbar, das zylindrische Gehäuse 44 in vorbestimmbare Vibrationen bzw. Schwingungen versetzt, woraufhin dann die Testperson geeignete Erkennungs- bzw. Bestätigungssignale durch Berühren zugehöriger Eingabeflächen auf der Bildschirmeinheit 10 abgibt.

Auch ist weiterbildungsgemäß vorgesehen, eine oder beide Betätigungs- und Sensoreinrichtungen bevorzugt randseitig mit LED-Anzeigen zu versehen, um ordnungsgemäßen Lade-, Betriebs- und/oder Verbindungszustand zum Hauptgehäuse 16 zu signalisieren. Auch ist in beiden zylindrischen Gehäusen 24, 44 eine nicht gezeigte wiederaufladbare Akku-Einheit vorgesehen, welche durch Aufsetzen auf das Aufsatz- bzw. Klappstück 40 in geeigneter Weise elektrisch kontaktiert und mit Ladestrom versehen wird.

Die vorliegende Erfindung ist nicht auf die dargestellte Ausführungsform beschränkt. So ist es insbesondere auch denkbar, andere Funktionalitäten in den gezeigten bi- oder multifunktionalen Betätigungs- und Sensoreinrichtungen vorzusehen, eine größere Anzahl derartiger Einheiten am bzw. im Gehäuse zu lagern, oder aber lediglich eine oder keine derartige Einheit vorzusehen.

Die Benutzerführung bzw. Darstellung von weiteren, ausschließlich mittels der Bildschirmeinheit zu realisierenden Funktionstests erfolgt analog der Darstellung gemäß gattungsbildender DE 200 15 449, ebenso wie weitere konstruktive bzw. die im Gehäuse 16 aufgenommene Datenverarbeitungsvorrichtung unmittelbar betreffende Details, welche weitgehend mit Hilfe herkömmlicher PC-Technologie realisierbar sind.

## Patentansprüche

1. Vorrichtung zur Vitalitätsdiagnostik an einer Testperson, mit
einer einer Datenverarbeitungsvorrichtung zugeordneten Ausgabeeinheit (10), die zum Ausgeben eines visuellen und/oder akustischen Signals für die Testperson ausgebildet ist,
sowie einer Betätigungs- und Sensoreinrichtung (10, 22, 42), die zum Erfassen einer Eingabebetätigung der Testperson als Reaktion auf das visuelle oder akustische Signal eingerichtet ist,
wobei ein Ausgangssignal der Betätigungs- und Sensoreinrichtung durch die Datenverarbeitungsvorrichtung datenmäßig erfasst und zur Ermittlung vitalitätsrelevanter Daten weiterverarbeitet wird
und die Betätigungs- und Sensoreinrichtung sowie die Ausgabeeinheit einen gemeinsamen berührungs- und/oder druckempfindlichen Bildschirm (10) aufweisen,
**dadurch gekennzeichnet, dass**
die Betätigungs- und Sensoreinrichtung mit mindestens einer zusätzlichen, manuell handhabbaren Detektor-und/oder Vibrationseinheit (22, 42) verbunden ist, die in einem per Signalübertragung drahtlos angebundenen Gehäuse (24, 44) mindestens zwei der folgenden Funktionalitäten zur Erzeugung des Ausgangssignals realisiert:
- Erfassen von auf die Detektoreinheit (22) wirkender Handkraft,
- Erfassen von durch Einblasen in die Detektoreinheit (42) erzeugten Luftdrucks,
- visuelles Darstellen und mittels eines einstellbaren Okulars (36) Verfremden eines durch die Testperson betrachtbaren optischen Musters,
- Erzeugen eines durch die Testperson zu ertastenden, in Frequenz oder Hub variabel steuerbaren Vibrationssignals,
wobei der Bildschirm und/oder die Detektor- und/oder Vibrationseinheit mit Mitteln zum Erfassen einer Einstell- oder Steuerbewegung (26) der Testperson versehen ist.

2. Vorrichtung nach Anspruch 1, **dadurch gekennzeichnet, dass** das optische Muster als aus einer Mehrzahl von Leuchtsegmenten zusammengesetzte, durch ein mittels eines Steuersignals der Datenverarbeitungsvorrichtung verstellbares Okular (36) betrachtbare Figur ausgebildet ist, wobei die Leuchtsegmente selektiv ansteuerbar ausgebildet und bevorzugt kreisförmig angeordnet sind.

3. Vorrichtung nach Anspruch 1 oder 2, **gekennzeichnet durch** eine am drahtlos angebundenen Gehäuse (24) extern vorgesehene Bügeleinheit (26), die zum Aufbringen einer zu messenden Handkraft **durch** die Testperson auf eine im Gehäuse (24) vorgesehene Druckerfassungseinheit (28) sowie zum Erzeugen von Bedien- und/oder Steuersignalen **durch** Druckbetätigung der Testperson ausgebildet ist.

4. Vorrichtung nach einem der Ansprüche 1 bis 3, **dadurch gekennzeichnet, dass** die manuell handhabbare Detektor-und/oder Vibrationseinheit (22, 42) mittels einer drahtlosen, auf Mikrowellen- oder Funkbasis wirkenden Verbindung mit der Gehäuseeinheit (16) verbunden ist.

5. Vorrichtung nach einem der Ansprüche 1 bis 4, **dadurch gekennzeichnet, dass** die Datenverarbeitungsvorrichtung zum Steuern der Betätigungs- und Sensoreinrichtung sowie der Ausgabeeinheit so ausgebildet ist, dass die Ausgabeeinheit auf einem Teilbereich des druckempfindlichen Bildschirms (10) ein visuell sichtbares Signal erzeugt und die Betätigungs- und Sensoreinrichtung zum Erfassen einer Berührung des Teilbereichs durch die Testperson ausgebildet ist.

6. Vorrichtung nach Anspruch 5, **dadurch gekennzeichnet, dass** die Datenverarbeitungsvorrichtung zum Erfassen und Auswerten einer Reihenfolge einer Berührung einer Mehrzahl von Teilbereichen als Reaktion auf das Anzeigen einer entsprechenden Mehrzahl von visuell sichtbaren Signalen auf dem Bildschirm ausgebildet ist.

7. Vorrichtung nach einem der Ansprüche 1 bis 6, **dadurch gekennzeichnet, dass** die Datenverarbeitungsvorrichtung zum Wiedergeben einer bevorzugt animierten, eine Diagnostikmaßnahme erläuternden Audio-, Text- und/oder Bilddarstellung zur Bedienerführung auf dem Bildschirm ausgebildet ist, die als Reaktion auf eine Berührung eines vorbestimmten Teilbereichs auf dem Bildschirm durch die Testperson aktivierbar ist.

8. Vorrichtung nach einem der Ansprüche 1 bis 7, **dadurch gekennzeichnet, dass** die Datenverarbeitungsvorrichtung Mittel zur Datenkommunikation mit einer über ein öffentliches Datenübertragungsnetz anbindbaren Servereinheit aufweist.

9. Vorrichtung nach einem der Ansprüche 1 bis 8, **dadurch gekennzeichnet**, der Bildschirm in einem Bildschirmgehäuse (12) gegenüber einer die Datenverarbeitungsvorrichtung enthaltenden Gehäuseeinheit (16) so klappbar gelagert ist, dass in einem Betriebszustand das Bildschirmgehäuse in mindestens einem vorbestimmten Winkel bevorzugt rastbar auf der Gehäuseeinheit aufsitzt und in einem Nicht-Betriebszustand das Bildschirmgehäuse mit seiner flachen Bildschirmseite auf einer Flachseite (20) der Gehäuseeinheit aufliegt.

10. Vorrichtung nach Anspruch 9, **dadurch gekennzeichnet, dass** die Gehäuseeinheit als flaches Gehäuse (16) ausgebildet ist, an welchem end- oder kantensextig eine schwenkbar mit dem Bildschirmgehäuse (12) verbundene Bügeleinheit (14) angelenkt ist.

11. Vorrichtung nach Anspruch 10, **dadurch gekennzeichnet, dass** dass die Bügeleinheit so ausgebildet ist, dass sie in dem Nicht-Betriebszustand auf einer Rückseite des Bildschirmgehäuses (12) aufliegt.

12. Vorrichtung nach einem der Ansprüche 1 bis 11, **dadurch gekennzeichnet, dass** die Detektor- und/oder Vibrationseinheit zumindest teilweise versendbar in einer Gehäuseeinheit (16) der Datenverarbeitungsvorrichtung vorgesehen ist.

## Claims

1. Device for vitality diagnostics on a test person having an output unit (10) associated with a data processing device which is constructed to output a visual and/or acoustic signal for the test person,
as well as an actuating and sensor device (10, 22, 42) which is set up to capture an input actuation of the test person in response to the visual or acoustic signal,
wherein an output signal of the actuating and sensor device is captured as data by the data processing device and processed further for determining vitality-related data
and the actuating and sensor device together with the output unit possess a common touch-sensitive and/or pressure-sensitive screen (10),
**characterised in that**
the actuating and sensor device is connected to at least one additional manually operable detector and/or vibration unit (22, 42) which in a housing (24, 44) wirelessly connected by signal transmission implements at least two of the following functionalities for generating the output signal:
- measurement of the manual force acting on the detector unit;
- measurement of air pressure produced by blowing into the detector unit (42);
- visual presentation and distortion by means of an adjustable eyepiece (36) of an optical pattern viewable by the test person;
- generation of a vibration signal variably controllable in frequency or amplitude;
wherein the screen and/or the detector and/or vibration unit is provided with means for detecting a setting or control movement (26) of the test person.

2. Device according to claim 1, **characterised in that** the optical pattern is constructed as a figure composed of a plurality of luminous segments viewable through an eyepiece (36) adjustable by means of a control signal of the data processing device, wherein the luminous segments are constructed to be selectively controllable and preferably arranged in circular shape.

3. Device according to claim 1 or 2, **characterised by** a handle unit (26) provided externally on the wirelessly linked housing (24) which is constructed for applying a manual force by the test person to be measured on a pressure measurement unit (28) provided in the housing (24) as well as for generating operating and/or control signals by pressure actuation on the part of the test person.

4. Device according to any of claims 1 to 3, **characterised in that** the manually operable detector and/or vibration unit (22, 42) is connected to the housing unit (16) by means of a wireless connection acting on the basis of microwaves or radio.

5. Device according to any of claims 1 to 4, **characterised in that** the data processing device for controlling the actuating and sensor device as well as the output unit are constructed so that the output unit produces a visually detectable signal on a subregion of the pressure-sensitive screen (10) and the actuating and sensor device is constructed for detecting touching of the subregion by the test person.

6. Device according to claim 5, **characterised in that** the data processing device is constructed for detecting and evaluating a series of touches of a plurality of subregions in response to the display of a corresponding plurality of visually detectable signals on the screen.

7. Device according to any of claims 1 to 6, **characterised in that** the data processing device is constructed for playing back a preferably animated audio, text and/or image presentation explaining a diagnostic measure for operator guidance on the screen which can be activated in response to touching of a predetermined subregion of the screen by the test person.

8. Device according to any of claims 1 to 7, **characterised in that** the data processing device has means for data communication with a server unit connectable via a public data transmission network.

9. Device according to one of the claims 1 to 8, **characterised in that** the screen is housed in a screen housing (12) for tilting relative a housing unit (16) containg the data processing device in such a way that in one operating condition the screen housing sits on the housing unit in at least one predetermined angle, preferably so as to be latchable, and in a non-operating condition the screen housing rests with its flat screen side on a flat side (20) of the housing unit.

10. Device according to claim 9, **characterised in that** the housing unit is constructed as a flat housing (16) on which on which at the end or edge a bracket unit (14) connected to the screen housing (12) is pivotably articulated.

11. Device according to claim 10, **characterised in that** the bracket unit is constructed so that in the non-operational state it lies on top of a rear side of the screen housing (12).

12. Device according to any of claims 1 to 11, **characterised in that** the detector and/or vibration unit is provided at least partially transmissibly in a housing unit (16) of the data processing device.

## Revendications

1. Dispositif de diagnostic de la vitalité sur une personne test, comportant:
- une unité de sortie (10), qui est associée à un organe de traitement de données et qui est réalisée pour délivrer un signal visuel et/ou acoustique à la personne test,
- ainsi qu'un organe d'actionnement et de détection (10, 22, 42), qui est agencé pour détecter une manoeuvre d'entrée de la personne test en réponse au signal visuel ou acoustique,
- un signal de sortie de l'organe d'actionnement et de détection étant enregistré comme donnée par l'organe de traitement des données et étant traité pour déterminer des données significatives sur la vitalité,
- et l'organe d'actionnement et de détection, ainsi que l'unité de sortie comportant un écran (10) commun, sensible au toucher et/ou sensible à la pression,
**caractérisé en ce que**:
- l'organe d'actionnement et de détection est relié à au moins une unité de détection et/ou de vibrations (22, 42) supplémentaire, actionnable manuellement, qui, à l'intérieur d'un boîtier (24, 44), relié sans fil pour la transmission de signal, remplit au moins deux des fonctionnalités suivantes pour générer le signal de sortie :
- détection d'une force manuelle exercée sur l'unité de détection (22),
- détection d'une pression d'air générée par insufflation dans l'unité de détection (42),
- représentation visuelle et, au moyen d'un oculaire (36) réglable, distanciation d'un modèle optique pouvant être observé par la personne test,
- génération d'un signal de vibration, apte à être commandé de manière variable en fréquence ou en amplitude et destiné à être perçu par la personne test,
- l'écran et/ou l'unité de détection et/ou de vibrations comportant des moyens pour détecter un mouvement de réglage ou de commande (26) de la personne test.

2. Dispositif selon la revendication 1, **caractérisé en ce que** le modèle optique est réalisé sous la forme d'une figure, formée par une pluralité de segments lumineux et pouvant être observée par un oculaire (36) réglable, au moyen d'un signal de commande de l'organe de traitement de données, les segments lumineux pouvant être activés sélectivement et étant agencés de préférence en forme de cercle.

3. Dispositif selon la revendication 1 ou 2, **caractérisé par** une unité à arceaux (26) qui est prévue à l'extérieur sur le boîtier (24) relié sans fil et qui est conçue pour l'application par la personne test d'une force manuelle à mesurer, sur une unité de détection de pression (28) prévue dans le boîtier (24) et pour la génération de signaux de manoeuvre et/ou de commande par une pression exercée par la personne test.

4. Dispositif selon l'une des revendications 1 à 3, **caractérisé en ce que** l'unité de détection et/ou de vibrations (22, 42) actionnable manuellement est reliée sans fil à l'unité de boîtier (16) via une liaison basée sur la transmission par micro-ondes ou par radio.

5. Dispositif selon l'une des revendications 1 à 4, **caractérisé en ce que** l'organe de traitement de données est réalisé pour commander l'organe d'actionnement et de détection, ainsi que l'unité de sortie, de telle sorte que l'unité de sortie génère un signal optiquement visible sur une zone partielle de l'écran tactile (10) et l'organe d'actionnement et de détection est réalisé pour détecter un contact exercé par la personne test sur la zone partielle.

6. Dispositif selon la revendication 5, **caractérisé en ce que** l'organe de traitement des données est réalisé pour enregistrer et analyser une succession de contacts sur une pluralité de zones partielles en réponse à l'affichage sur l'écran d'une pluralité correspondante de signaux optiquement visibles.

7. Dispositif selon l'une des revendications 1 à 6, **caractérisé en ce que** l'organe de traitement de données est réalisé pour reproduire sur l'écran une représentation, sous forme audio, de message écrit et/ou de graphique, de préférence animée, expliquant une disposition de diagnostic et destinée à guider l'utilisateur, laquelle représentation peut être activée en réponse à un contact d'une zone partielle prédéterminée, exercé par la personne test sur l'écran.

8. Dispositif selon l'une des revendications 1 à 7, **caractérisé en ce que** l'organe de traitement des données comporte des moyens pour la communication des données à une unité de serveur susceptible d' être raccordée par l'intermédiaire d'un réseau public de transmission de données.

9. Dispositif selon l'une des revendications 1 à 8, **caractérisé en ce que** l'écran est monté dans un boîtier d'écran (12), apte à être rabattu par rapport à une unité de boîtier (16) contenant l'organe de traitement de données, de telle sorte que, dans une position de service, le boîtier d'écran soit relevé sur l'unité de boîtier, de préférence de manière apte à être bloqué selon au moins un angle prédéterminé, et que le boîtier d'écran, dans une position hors service, repose par sa face d'écran plane sur une face plane (20) de l'unité de boîtier.

10. Dispositif selon la revendication 9, **caractérisé en ce que** l'unité de boîtier est réalisée sous la forme d'un boîtier plat (16), contre l'extrémité ou le bord duquel est articulée une unité à arceaux (14), reliée de manière pivotante au boîtier d'écran (12).

11. Dispositif selon la revendication 10, **caractérisé en ce que** l'unité à arceaux est réalisée de telle sorte que, dans la position hors service, elle repose sur une face arrière du boîtier d'écran (12).

12. Dispositif selon l'une des revendications 1 à 11, **caractérisé en ce que** l'unité de détection et de vibrations est prévue au moins partiellennent escamotable dans une unité de boîtier (16) du dispositif de traitement des données.
